(19) **Europäisches Patentamt / European Patent Office / Office européen des brevets**

(11) **EP 2 764 365 B1**

(12) # FASCICULE DE BREVET EUROPEEN

(45) Date de publication et mention de la délivrance du brevet:
**02.11.2016 Bulletin 2016/44**

(21) Numéro de dépôt: **12780222.1**

(22) Date de dépôt: **03.10.2012**

(51) Int Cl.:
*G01N 33/574* (2006.01)

(86) Numéro de dépôt international:
**PCT/FR2012/052242**

(87) Numéro de publication internationale:
**WO 2013/050705 (11.04.2013 Gazette 2013/15)**

(54) **PROCÉDÉ D'IDENTIFICATION D'UN CANCER AGRESSIF ET/OU SUSCEPTIBLE DE DÉVELOPPER DES MÉTASTASES**

VERFAHREN ZUR IDENTIFIZIERUNG VON AGGRESSIVEM UND/ODER WAHRSCHEINLICH METASTASEN ENTWICKELNDEM KREBS

METHOD FOR IDENTIFYING CANCER THAT IS AGGRESSIVE AND/OR LIKELY TO DEVELOP METASTASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.10.2011 FR 1158910**

(43) Date de publication de la demande:
**13.08.2014 Bulletin 2014/33**

(73) Titulaires:
- **Université Claude Bernard Lyon I**
  **69622 Villeurbanne Cedex (FR)**
- **Centre National de la Recherche Scientifique**
  **75794 Paris Cedex 16 (FR)**
- **Centre Léon Bérard**
  **69373 Lyon Cedex 08 (FR)**

(72) Inventeurs:
- **MOYRET-LALLE, Caroline**
  **F-69530 Brignais (FR)**
- **TREILLEUX, Isabelle**
  **F-69004 Lyon (FR)**
- **LEON-GODDARD, Sophie**
  **F-01500 Chateau-Gaillard (FR)**
- **COX, David**
  **F-69008 Lyon (FR)**

(74) Mandataire: **Sarlin, Laure V.**
**Cabinet Beau de Loménie**
**51, avenue Jean-Jaurès**
**BP 7073**
**69301 Lyon Cedex 07 (FR)**

(56) Documents cités:
- CHAN ANNA H ET AL: "Molecular cloning and characterization of a RING-H2 finger protein, ANAPC11, the human homolog of yeast Apc11p", JOURNAL OF CELLULAR BIOCHEMISTRY, vol. 83, no. 2, 2001, pages 249-258, XP002676632, ISSN: 0730-2312
- COMBES J D ET AL: "Contribution of cell culture, RNA extraction, and reverse transcription to the measurement error in quantitative reverse transcription polymerase chain reaction-based gene expression quantification", ANALYTICAL BIOCHEMISTRY, ACADEMIC PRESS INC, NEW YORK, vol. 393, no. 1, 1 octobre 2009 (2009-10-01), pages 29-35, XP026419318, ISSN: 0003-2697 [extrait le 2009-06-13]
- Moyret-Lalle caroline: "Analysis of APC/C key element in breast and colon cancer reveals APC11 subunit expression as a cancer aggressiveness marker.", , 27 juillet 2009 (2009-07-27), XP002676633, Extrait de l'Internet: URL:http://liveweb.archive.org/http://ispb .univ-lyon1.fr/recherche/aeres/MOYRET%20LA LLE%20C.pdf [extrait le 2012-05-24]
- CHEN CESHI ET AL: "Genetic and expression aberrations of E3 ubiquitin ligases in human breast cancer", MOLECULAR CANCER RESEARCH, vol. 4, no. 10, octobre 2006 (2006-10), pages 695-707, XP002676634, ISSN: 1541-7786

SEQUENCE LISTING

<110> UNIVERSITE CLAUDE BERNARD LYON I
CENTRE NATIONAL DE LA RECHERCHE SCIENTIFIQUE
CENTRE LEON BERARD
MOYRET-LALLE, Caroline
TREILLEUX, Isabelle
LEON-GODDARD, Sophie
COX, David

<120> PROCEDE D'IDENTIFICATION D'UN CANCER AGRESSIF ET/OU SUSCEPTIBLE DE DEVELOPPER DES METASTASES

<130> 1H708400 0160 WO PCT

<150> FR 11 58910
<151> 2011-10-03

<160> 2

<170> PatentIn version 3.3

<210> 1
<211> 13
<212> PRT
<213> Homo sapiens

<400> 1

```
        Met Lys Val Lys Ile Lys Cys Trp Asn Gly Val Ala Thr
        1                   5                   10
```

<210> 2
<211> 16
<212> PRT
<213> Homo sapiens

<400> 2

```
    Ala Asn Asp Glu Asn Cys Gly Ile Cys Arg Met Ala Phe Asn Gly Cys
    1               5                   10                  15
```

**Description**

La présente invention concerne le domaine médical en général, et en particulier du cancer.

Plus précisément, l'invention concerne un procédé et un kit de détermination du caractère invasif et/ou de la susceptibilité de développer des métastases chez des patients atteints de cancer, et en particulier de cancer du sein ou de cancer du côlon.

Le cancer constitue un problème majeur de santé publique. Des efforts importants ont été apportés concernant le diagnostic des cancers, et en particulier à des stades précoces de la maladie. Une fois le cancer diagnostiqué, il reste néanmoins important de pouvoir disposer de marqueurs permettant de déterminer le pronostic, bon ou mauvais, chez les patients atteints de cancer, en particulier pour adapter le traitement et/ou le suivi des patients. Par exemple, les patients atteints d'un cancer associé à un mauvais pronostic seront des candidats pour la mise en place d'une thérapie plus agressive, afin d'essayer de traiter préventivement le patient avant l'apparition de métastases, comme par exemple le traitement par le trastuzumab des patientes atteintes de cancer du sein présentant une surexpression de la protéine HER2 mise en évidence par immunohistochimie.

Parmi les exemples de critères permettant de définir un pronostic, le caractère invasif c'est-à-dire agressif du cancer, et en particulier le risque d'invasion métastatique à distance, représentent des cibles majeures permettant d'orienter la thérapeutique des patients atteints de cancer.

Un certain nombre de marqueurs cliniques et histologiques, dont des marqueurs protéiques, existent à ce jour et permettent de déterminer le pronostic d'un cancer (i.e. cancer de bon ou de mauvais pronostic), tels que l'âge du patient, la taille de la tumeur, l'envahissement ganglionnaire (absence vs. présence et/ou degré d'envahissement). Plus spécifiquement pour les cancers du sein sont utilisés le grade histopronostique de Scarff Bloom et Richardson (SBR) qui comprend trois grades I II III, obtenu par l'addition de trois critères : architecture, atypies cytonucléaires et nombre de mitoses (les trois critères étant cotés en 1, 2 et 3), l'expression des récepteurs hormonaux (récepteurs aux oestrogènes et récepteurs à la progestérone) et l'expression de l'oncogène HER2. Plus spécifiquement pour les cancers du côlon, le critère pronostique le plus important est le stade de la maladie selon la classification TNM (*Tumor, Nodes, Metastasis*) où ces trois critères - taille et profondeur de la tumeur, atteinte ou non des ganglions lymphatiques et nombre de ganglions atteints, présence ou non de métastases - permettent de définir le stade du cancer selon la classification TNM. Le stade des cancers colorectaux au moment du diagnostic est généralement exprimé par un chiffre romain allant de 0 à IV (selon la classification TNM). Deux critères permettent de considérer ces cancers comme de bon pronostic : un histologique (inflammation intratumorale ou péritumorale avec follicules lymphoïdes) et un génétique (le génotype MSI (*Microsatellite Instability*)). Des facteurs prédictifs de la réponse au traitement pour ces cancers sont par exemple le génotype MSI (*Microsatellite Instability*), les mutations K-RAS et EGFR (*epidermal growth factor receptor*).

La publication Combes J. D et al., (Contribution of cell culture, RNA extraction, and reverse transcription to the measurement error in quantitative reverse transcription polymerase chain reaction-based gene expression quantification, Analytical Biochemistry, Academic Press Inc, New York, vol. 393, no.1, 2009, 29-35 discute de l'importance des ubiquitines de type E3 dans le cancer du sein.

Il existe également des marqueurs pronostics basés sur l'analyse de l'expression de plusieurs gènes (analyses transcriptomiques, exemple : signature d'expression de 70 gènes pour les cancers du sein (MammaPrint(R) (Agendia, Amsterdam,NL). Cependant, ces analyses restent longues, assez coûteuses et peuvent s'avérer complexes, voire parfois inexactes. Par exemple, une étude publiée par le groupe de Catherine Hill (Michiels S., et coll., 2005, Lancet, 365(9458):488-492) montre que les listes de gènes identifiés comme pronostiques à partir d'études transcriptomiques sont très instables d'une étude à l'autre; les signatures moléculaires étant très dépendantes de la sélection des patients. 5/7 analyses transcriptomiques qu'ils ont étudiées classent les patients de façon aléatoire. Une autre étude *(Washington University School of Medicine,* St. Louis) portant sur le sous-type « *normal-like* » dans les cancers du sein montre qu'une signature de 50 gènes, en analyse transcriptomique, avait permis de mettre en évidence ce 5ème sous-type de cancers du sein. Cependant, les chercheurs ont montré qu'au lieu d'être le 5ème sous-type de cancers du sein, la classification "normal-like" est un indicateur de contamination d'un échantillon tumoral par du tissue normal.

Cependant, il existe un réel besoin de disposer de marqueurs pronostics concernant l'agressivité, et en particulier la progression métastatique des cancers. C'est dans ce contexte que la présente invention se propose de fournir un nouveau biomarqueur pronostic, en particulier de l'agressivité, et notamment du risque de progression métastatique, dans les cancers du sein et du côlon. De plus, la présente invention trouve une application toute particulière pour le pronostic des cancers du sein considérés au moment du diagnostic comme de bon pronostic sur la base des critères classiques actuellement disponibles, et notamment considérés comme non métastatiques au diagnostic, en particulier sur la base du grade SBR, des récepteurs hormonaux.

S'agissant des cancers du côlon, la présente invention permet également d'établir un pronostic par rapport à l'apparition d'une récidive, qu'elle soit locale ou « à distance », sans lien avec le statut d'envahissement ganglionnaire.

Selon un premier aspect, la présente invention a donc pour objet un procédé d'identification d'un cancer agressif et/ou susceptible de développer des métastases chez un patient atteint de cancer du sein ou du côlon, de préférence de cancer du sein, comprenant les étapes consistant à :

- mesurer le niveau d'expression ou l'activité de la protéine APC11 dans un échantillon issu dudit patient, et
- classer le cancer comme agressif et/ou susceptible de développer des métastases lorsqu'une différence significative du niveau d'expression ou d'activité de la protéine APC11 est mise en évidence par rapport à une valeur de référence telle que définie à la revendication 1.

Ainsi, les inventeurs ont montré que la protéine APC11 (*Anaphase Promoting Complex subunit 11*) est un marqueur pronostic de l'agressivité et/ou du développement des métastases pour les cancers du sein ou du côlon. Le procédé selon l'invention est donc un procédé pronostic des cancers du sein et du côlon, de préférence des cancers du sein.

La protéine APC11 est une ubiquitine ligase de type E3 qui possède un rôle central dans la régulation du cycle cellulaire.

La mesure du niveau d'expression ou de l'activité enzymatique de la protéine APC11 selon l'invention permet en particulier d'établir un pronostic pour les cancers du sein, et tout particulièrement pour des cancers du sein qui ont été considérés comme de bon pronostic au moment du diagnostic, et notamment considérés comme non métastatiques au diagnostic. Par « cancers du sein considérés comme de bon pronostic », on entend par exemple des cancers de petite taille (< 15 mm), de grade SBR 1 et 2, ayant des récepteurs hormonaux positifs (et notamment plus de 10 % des cellules tumorales exprimant les récepteurs hormonaux en analyse immunohistochimique), et sans surexpression de Her2. Par « cancers du sein considérés comme non métastatiques», on entend sans métastases à distance, y compris sus-claviculaires.

S'agissant du caractère agressif des cancers du côlon, l'invention permet tout particulièrement d'établir un pronostic par rapport à l'apparition d'une récidive locale encore nommée maladie résiduelle, c'est-à-dire un pronostic sur la réapparition d'une tumeur au même site ou le grossissement de la tumeur existant auparavant au même site et qui n'aurait pas totalement disparue. Ainsi, l'invention présente donc un intérêt particulier dans le cas de résistance au traitement puisque les récidives locales sont le plus souvent liées à une résistance au traitement.

Par ailleurs, s'agissant toujours des cancers du côlon, la présente invention permet également d'établir un pronostic par rapport à l'apparition d'une récidive « à distance », c'est-à-dire par rapport à la susceptibilité de développer des métastases, qui sont le plus souvent dans les poumons ou le foie dans ce type de cancer.

En conséquence, la présente invention permet tout particulièrement d'établir un pronostic pour les cancers du côlon en lien avec les deux notions de récidive, à la fois récidive locale et récidive « à distance » ou métastases, et ce quel que soit le statut d'envahissement ganglionnaire.

De manière préférée, l'invention permet d'établir un pronostic pour les cancers du côlon sans envahissement ganglionnaire.

Dans le cadre de la présente demande, les termes « cancer » et « tumeur » sont utilisés indifféremment pour désigner un cancer, c'est-à-dire qu'ici le terme « tumeur » s'entend désigner une tumeur maligne c'est-à-dire cancéreuse.

La mise en évidence d'une différence significative du niveau d'expression ou de l'activité de la protéine APC11 mesuré dans l'échantillon issu du patient à tester par rapport à une valeur de référence est associée à un phénotype agressif, et/ou à un risque accru de métastases, et ainsi à un mauvais pronostic pour ledit patient. De préférence, la protéine APC11 dont l'expression ou l'activité est mesurée dans le cadre du procédé de l'invention est la protéine APC11 humaine, en particulier telle que décrite par Chan, AH et coll., 2001, J. Cell Biochem., 83 : 249-258 ou bien celle dont la séquence est enregistrée dans la base de données GenBank: AAH00607.2 GI:51574060 (18 mars 2009).

La détection de la protéine APC11 peut être réalisée par n'importe quelle technique classique bien connue de l'homme du métier, parmi lesquelles on peut notamment citer le western blot, l'immunohistochimie, l'immunofluorescence, l'immunoprécipitation, la technique ELISA, etc.

De manière préférée, la protéine APC11 est détectée à l'aide d'anticorps anti-APC11, et notamment d'anticorps monoclonaux anti-APC11 obtenus à l'aide de la partie N-terminale de APC11, comprenant au moins une partie du domaine RING-finger indispensable à l'ubiquitinylation, comme par exemple à l'aide des peptides de séquence MKVKIKCWNGVAT (SEQ ID NO : 1) ou de séquence ANDENCGICRMAFNGC (SEQ ID NO : 2).

De préférence, la protéine APC11 est détectée par immunohistochimie à l'aide d'anticorps anti-APC11 et notamment d'anticorps monoclonaux anti-APC11 obtenus à l'aide de la partie N-terminale de APC11, comprenant au moins une partie du domaine RING-finger indispensable à l'ubiquitinylation, comme par exemple à l'aide des peptides de séquence SEQ ID NO : 1 ou SEQ ID NO : 2.

Une fois détectée, le niveau d'expression de la protéine APC11 peut également être quantifié par toute méthode de quantification ou semi-quantification classiquement utilisée dans le domaine, parmi lesquelles on peut notamment citer le western blot, la technique ELISA, etc. En particulier, la détermination du niveau d'expression ou de l'activité de la protéine est réalisée à l'aide d'outils ou de réactifs qui sont spécifiques de la protéine et qui permettent, directement ou indirectement de quantifier son niveau d'expression, comme par exemple des anticorps anti-APC11. De préférence, la quantification du niveau d'expression de la protéine APC11 est réalisée par western blot, par immunohistochimie, ou encore par immunofluorescence.

Il est également possible de déterminer le pronostic selon l'invention à partir de la mesure de l'activité enzymatique de la protéine APC11. Parmi les méthodes de mesure qui peuvent être utilisées, on peut notamment citer la quantification de protéines ubiquitinylées, par exemple par Western Blot à l'aide d'un anticorps anti-ubiquitine, ou bien toute méthode de mesure d'une activité enzymatique classiquement utilisée dans le domaine et bien connue de l'homme du métier, telle que la mesure *in vitro* de l'activité ubiquitine ligase.

Au sens de la présente invention, on entend par « échantillon issu du patient à tester » ou « échantillon à tester », tout échantillon biologique susceptible de contenir la protéine APC11 et provenant du patient chez qui on souhaite déterminer l'agressivité du cancer et/ou sa susceptibilité de développer des métastases.

Par conséquent, l'échantillon à tester utilisé dans le cadre du procédé selon l'invention peut être de différente nature. En particulier, l'échantillon à tester est choisi parmi les tissus de tumeurs du sein ou de tumeurs coliques, ou encore les cellules isolées à partir de ces tissus.

De préférence, l'échantillon à tester issu dudit patient est un tissu, et en particulier un tissu qui est issu de la tumeur primaire, du sein ou du côlon.

Le niveau d'expression ou l'activité de la protéine APC11 qui est détectée dans l'échantillon à tester est alors comparé à une valeur de référence.

Dans le cadre de la présente invention, la valeur de référence peut correspondre à une valeur seuil d'expression ou d'activité ou bien au niveau d'expression ou d'activité de la protéine APC11 mesurée dans un échantillon biologique issu d'un sujet sain, en particulier non atteint de cancer, ou bien atteint du même type de cancer mais qui n'est pas agressif ni susceptible de métastaser. La valeur de référence peut également correspondre à une valeur moyenne du niveau d'expression ou d'activité de la protéine APC11 qui est détecté sur un pool d'échantillons tels que définis ci-dessus.

Les échantillons biologiques à partir desquels peut être déterminée la valeur de référence peuvent être de différentes natures et notamment choisi parmi les échantillons biologiques qui sont mentionnés ci-dessus s'agissant de l'échantillon à tester (tissu, cellules, etc). Avantageusement, ces échantillons biologiques sont de même nature que celle de l'échantillon biologique à tester ou tout du moins d'une nature compatible pour constituer une référence quant à la détection de l'expression de la protéine APC11. En particulier, ces échantillons sont de préférence issus de personnes ayant les mêmes caractéristiques ou une majorité de caractéristiques communes, notamment du même sexe et/ou d'un âge similaire ou identique et/ou de même origine ethnique, avec celles du patient chez qui on souhaite déterminer l'agressivité du cancer, et en particulier sa susceptibilité de développer des métastases. Par exemple, de tels échantillons sont choisis parmi des tissus provenant de réductions mammaires, ou de tissus normaux de côlon prélevés à distance du tissu tumoral.

Dans le cas d'une comparaison avec une valeur seuil, cette valeur peut être obtenue en déterminant le nombre ou la proportion, par exemple un pourcentage, de cellules qui expriment la protéine APC11 ou encore la quantité ou l'intensité d'expression ou d'activité de la protéine APC11 au sein d'échantillons issus de patients atteints de cancer du sein ou du côlon et dont on connait le sort ultérieur s'agissant de l'agressivité du cancer, et/ou du développement de métastases (instabilité chromosomique vs instabilité microsatellitaire pour les cancers du côlon MSI (*Microsatellite Instability*), patients présentant une rechute après traitement, etc). Par exemple, la valeur seuil peut correspondre au pourcentage de cellules qui expriment la protéine APC11 détectée notamment par immunohistochimie sur des coupes de tissus de patients atteints de cancer du sein ou du côlon, et dont on connait le sort ultérieur s'agissant de l'agressivité du cancer, et/ou du développement de métastases. Dans ce cas, ce pourcentage peut notamment être estimé en microscopie photonique classique par comptage des cellules positives pour l'expression ou l'activité d'APC11 par rapport aux cellules négatives dans un champ donné. Un exemple de valeur seuil peut correspondre à 50 % de cellules positives pour la protéine APC11 détectée par immunohistochimie sur coupes de tissus de patients atteints de cancer du sein. La valeur seuil peut également correspondre à une quantité de protéine APC11 mesurée par western blot dans un échantillon de cellules provenant de patients atteints de cancer du sein ou du côlon dont on connaît le sort ultérieur.

Ainsi, avant la mesure du niveau d'expression ou d'activité de la protéine APC11 proprement dite dans l'échantillon à tester, le procédé de l'invention peut comprendre l'obtention préalable de la valeur de référence à laquelle le niveau d'expression qui sera détecté dans l'échantillon à tester pourra être comparé afin de déterminer s'il existe ou non une différence significative du niveau d'expression ou d'activité de la protéine APC11 dans l'échantillon à tester.

Selon l'invention, la mise en évidence d'une différence significative du niveau d'expression ou d'activité de la protéine APC11 dans l'échantillon à tester par rapport à la valeur de référence est associée à l'identification d'un cancer agressif et/ou susceptible de développer des métastases, et permet ainsi de classer le cancer dont est atteint le patient testé comme un cancer de mauvais pronostic.

Par « différence significative du niveau d'expression de la protéine APC11 », on entend aussi bien une diminution qu'une augmentation significative du niveau d'expression de la protéine APC11.

Dans le cadre de l'invention, une diminution définit à la fois la mesure d'un niveau significativement inférieur dans l'échantillon à tester par rapport à la valeur de référence ou bien une absence dans l'échantillon à tester.

Ainsi, et comme mentionné ci-dessus, la détection au sens de l'invention peut aussi bien revêtir un aspect qualitatif que quantitatif.

Selon un mode de réalisation particulièrement avantageux, le procédé de l'invention permet, chez un patient atteint de cancer du sein, de classer le cancer comme susceptible de développer des métastases lorsqu'une diminution significative du niveau d'expression ou de l'activité de APC11, de préférence du niveau d'expression, est mise en évidence dans l'échantillon issu du patient à tester par rapport à la valeur de référence. De préférence, une telle diminution est d'au moins 15 %, d'au moins 25 %, d'au moins 50 %, d'au moins 75 %, de 100 % de l'expression ou de l'activité de APC11, de manière encore préférée d'au moins 50 % par rapport à la valeur de référence. En particulier, le procédé de l'invention permet de classer un cancer du sein comme susceptible de développer des métastases lorsque moins de 50 % des cellules expriment la protéine APC11, de préférence mise en évidence par immunohistochimie à l'aide d'anticorps anti-APC11.

Le procédé de l'invention permet de manière avantageuse d'identifier un cancer du sein susceptible de développer des métastases chez une patiente atteinte d'un cancer du sein qui avait été considérée comme de bon pronostic au moment du diagnostic, et en particulier considéré comme non susceptible de développer des métastases. Le procédé de l'invention permet également de considérer une patiente comme atteinte d'un cancer du sein susceptible de développer des métastases indépendamment du statut des récepteurs hormonaux de cette patiente, c'est-à-dire que celui-ci soit positif ou négatif. En particulier, la présente invention permet d'identifier un cancer du sein susceptible de développer des métastases chez un patient atteint d'un cancer du sein de type luminal A, qui est un type de cancer caractérisé par l'expression positive des récepteurs hormonaux (récepteurs aux oestrogènes et/ou récepteurs à la progestérone) et considéré comme de bon pronostic.

Selon un autre mode de réalisation préféré, le procédé de l'invention permet, chez un patient atteint de cancer du côlon, de classer le cancer comme agressif lorsqu'une augmentation significative du niveau d'expression ou de l'activité de la protéine APC11, de préférence du niveau d'expression, est mise en évidence dans l'échantillon issu du patient à tester par rapport à la valeur de référence. De préférence, une telle augmentation est d'au moins 15 %, d'au moins 25 %, d'au moins 50 %, d'au moins 75 %, de 100 % de l'expression d'APC11, de manière encore préférée d'au moins 50 %, par rapport à la valeur de référence.

Le procédé de l'invention permet de manière avantageuse d'identifier un cancer du côlon agressif chez un patient lorsque ledit patient est atteint d'un cancer du côlon sans envahissement ganglionnaire.

Tous les modes de réalisation préférés qui sont mentionnés ci-dessus peuvent être combinés indifféremment entre eux dans le cadre de l'invention.

Selon un deuxième aspect, la présente invention a également pour objet l'utilisation de la mesure du niveau d'expression ou de l'activité d'APC11 en tant que biomarqueur pronostic des cancers du sein et du côlon.

Selon un mode de réalisation avantageux de l'invention, la mesure du niveau d'expression ou de l'activité d'APC11 est utilisée de préférence en tant que biomarqueur pronostic des cancers du sein, et en particulier des cancers du sein considérés comme de bon pronostic au moment du diagnostic, et de manière encore préférée considérés comme non métastatiques au diagnostic.

En particulier, le pronostic consiste à déterminer l'agressivité et/ou la susceptibilité de développer des métastases des cancers du sein et du côlon, et en particulier de la susceptibilité de développer des métastases des cancers du sein ou de la susceptibilité de développer des métastases des cancers du côlon, c'est-à-dire du risque de récidive « à distance » des cancers du côlon.

Selon un autre mode de réalisation préféré, le pronostic consiste à déterminer le risque de récidive des cancers du côlon, et en particulier le risque de récidive locale.

Dans le cadre de ce deuxième aspect, l'invention concerne de préférence l'utilisation de la mesure du niveau d'expression de la protéine APC11.

Tous les modes de réalisation préférés et leurs combinaisons qui sont mentionnés ci-dessus concernant le procédé constituent également des modes de réalisation préférés s'agissant de l'utilisation.

Selon un troisième aspect, la présente invention a aussi pour objet un kit d'identification d'un cancer agressif et/ou susceptible de développer des métastases chez un patient atteint de cancer du sein ou du côlon comprenant un ou plusieurs réactifs spécifiques permettant de mesurer le niveau d'expression ou l'activité de la protéine APC11 et un échantillon contrôle.

De préférence, le kit de l'invention contient des anticorps anti-APC11 en tant que réactif spécifique, et en particulier des anticorps monoclonaux anti-APC11, notamment des anticorps monoclonaux anti-APC11 obtenus à l'aide de la partie N-terminale de APC11, comprenant au moins une partie du domaine RING-finger indispensable à l'ubiquitinylation, comme par exemple à l'aide des peptides de séquence SEQ ID NO : 1 ou SEQ ID NO : 2.

L'échantillon contrôle peut être un contrôle positif ou un contrôle négatif. Parmi les exemples d'échantillon contrôle positif, on peut notamment citer un échantillon issu d'un patient atteint de cancer du sein ou du côlon qui présente un caractère agressif et/ou qui a développé des métastases, ou bien un contrôle négatif, comme par exemple un échantillon issu d'un sujet sain. Pour le contrôle positif, il peut également s'agir d'un échantillon calibré pour contenir la quantité de protéine APC11 qui correspond à la quantité moyenne mesurée dans un pool d'échantillons de patients atteints de cancer du sein ou du côlon qui présente un caractère agressif et/ou qui a développé des métastases. De même, pour le contrôle négatif, il peut également s'agir d'un échantillon calibré pour contenir la quantité de protéine APC11 qui correspond à la quantité moyenne mesurée dans un pool d'échantillons de sujets sains.

De préférence, le kit peut également contenir à la fois un contrôle positif et un contrôle négatif, et notamment choisis chacun parmi les exemples précités.

Le kit de l'invention peut en outre contenir tout autre réactif ou dispositif nécessaire pour réaliser le procédé selon l'invention, comme par exemple, des anticorps secondaires notamment biotinylés, des réactifs de fixation spécifique comme par exemple le paraformaldéhyde, le Bouin Hollande, le méthanol, etc ; des réactifs d'amplification spécifique comme par exemple un complexe streptavidinebiotine, etc ; des réactifs de révélation avec un chromogène spécifique comme par exemple la DAB (Diaminobenzidine), etc ; des réactifs de contre-coloration comme le bluing, l'hématoxyline, etc.

L'invention couvre également l'utilisation d'un kit selon l'invention pour réaliser le procédé de l'invention, et en particulier pour identifier un cancer agressif et/ou susceptible de développer des métastases chez un patient atteint de cancer du sein ou du côlon, de préférence de cancer du sein, et de manière particulièrement préférée chez un patient atteint d'un cancer du sein considéré comme de bon pronostic au moment du diagnostic, et notamment considéré comme non métastatique au diagnostic. La présente invention couvre également de manière préférée, l'utilisation d'un kit selon l'invention pour identifier un cancer agressif et/ou susceptible de développer des métastases chez un patient atteint de cancer du côlon, et en particulier pour identifier un risque de récidive, que ce soit de récidive locale ou de récidive « à distance ».

Tous les modes de réalisation préférés et leurs combinaisons qui sont mentionnés ci-dessus concernant le procédé et l'utilisation constituent également des modes de réalisation préférés s'agissant du kit et de son utilisation.

Diverses autres caractéristiques ressortent de la description faite ci-dessous en référence aux dessins annexés qui montrent, à titre d'exemples non limitatifs, des formes de réalisation de l'objet de l'invention.

La **figure 1** représente l'expression de APC11 mise en évidence par RT-PCR **(A)** et Western Blot **(B)** dans différentes lignées cellulaires de cancer du côlon.

La **figure 2** représente l'expression de APC11 mise en évidence par RT-PCR **(A)** et Western Blot **(B)** dans différentes lignées cellulaires de cancer du sein (M+ : métastatique, $M_0$ : non métastatique, E : phénotype épithélial, M : phénotype mésenchymateux, E-M phénotype mixte épithélial-mésenchymateux, et la quantification de l'expression de la protéine APC11 à partir des résultats de Western Blot **(C).**

La **figure 3** représente l'expression de APC11 mise en évidence par RT-PCR dans des échantillons de tumeur primaire de cancer du sein.

La **figure 4** représente l'expression de la protéine APC11 en immunohistochimie sur des coupes de tissu mammaire normal **(A)** et de tumeurs du sein **(B, C).** Magnitude X 40.

La **figure 5** est une courbe de survie sans progression métastatique (Kaplan Meier) établie en fonction du pourcentage de cellules marquées pour la protéine APC11, la valeur seuil est de 50 % de cellules marquées. La courbe représente la probabilité de survie en fonction du temps.

La **figure 6** est un histogramme qui représente la répartition du pourcentage de cellules marquées pour APC11 sur l'ensemble des patients atteints de tumeurs du côlon analysés en TMA.

La **figure 7** est un histogramme qui représente la répartition de l'âge sur l'ensemble des patients atteints de tumeurs du côlon analysés en TMA.

La **figure 8** est un histogramme qui représente la répartition **(A)** des 4 stades de la pathologie tumorale ou **(B)** des patients avec récidive (1) ou sans récidive (2) locale sur l'ensemble des patients atteints de tumeurs du côlon analysés en TMA.

La **figure 9** est un histogramme qui représente la répartition de l'envahissement ganglionnaire sur l'ensemble des patients atteints de tumeurs du côlon analysés en TMA.

La **figure 10** est un histogramme qui représente la répartition de l'envahissement métastatique sur l'ensemble des patients atteints de tumeurs du côlon analysés en TMA.

**EXEMPLES**

**Matériels et Méthodes**

*Cellules*

*Lignées cellulaires de cancer du sein*

Sauf mention contraire, les lignées cellulaires établies de cancer du sein ont été obtenues de l'ATCC (American Type Culture Collection, www.ATCC.org).

*Lignées cellulaires de cancer du côlon*

Sauf mention contraire, les lignées cellulaires établies de cancer du côlon ont été obtenues de l'ATCC (American Type Culture Collection, www.ATCC.org).

La lignée colorectale EB a été fournie par P. Shaw et les cellules Co-115, IS1 (Isreco1), IS2, IS3, TC7 et TC71 ont été fournie par R. Hamelin. La lignée cellulaire IS2 est dérivée des métastases hépatiques d'un adénocarcinome colique (Gayet et al., 2001, Oncogene, 20(36) :5025-5032; Cajot et al., 1997, Cancer Res., 57(13) : 2593-2597. Les autres lignées cellulaires sont dérivées de carcinomes du côlon primaires humains.

Les cellules HME-1 (Cambrex, Clonetics) ont été cultivées en MEGM à 37°C avec $CO_2$ comme décrit par Stampfer, (Hammond et Stampfer, 1984, Proc Natl Acad Sci U S A., ;81(17):5435-5439.).Les cellules ont été cultivées selon les protocoles de l'ATCC à 90 % de confluence et ont été collectées pour la préparation d'ARN totaux ou de protéines.

*Extraction des ARN totaux et synthèse des ADNc*

Les ARN totaux provenant de deux échantillons de tissus normaux mammaires humains et deux échantillons de tissus normaux de colon humains ont été obtenus auprès de l'Institut BioChain (BioChain Institute Inc., USA). Les ARN totaux ont été isolés à l'aide du réactif Tri-Reagent (Sigma Chemical Co.). Une étape supplémentaire de purification (vis à vis de contamination par l'ADN) a été réalisée à l'aide de tubes Phase-Lock gel (Eppendorf). La concentration en ARN des échantillons a été déterminée par spectrophotométrie par lecture de l'absorbance à 260 nm à l'aide du spectrophotomètre Beckman UV-VIS. La qualité des ARN a été analysée sur gel d'agarose 1% MOPS 1X. Les ADNc ont été synthétisés à l'aide du kit first-strand cDNA synthesis (Amersham Pharmacia Biotech) selon les recommandations du fabricant.

*PCR quantitative en temps réel*

L'expression de l'ARNm d'APC11 a été quantifiée avec la technique « two-step real-time PCR » à l'aide de l'appareil LightCycler (Roche Molecular Biochemicals). Les primers utilisés dans la réaction de PCR étaient disponibles dans le commerce (Applied Biosystems). Toutes les amplifications ont été réalisées en capillaires (Roche) avec le kit « LightCycler FastStart DNA Master PLUS SYBR Green I ». Chaque réaction contient de la polymerase FastStart TaqDNA polymerase, du tampon de réaction, un mix de deoxynucléosides, du MgCl2 inclus dans le kit, 0.25 $\mu$M de chaque primer, et 1,67 $\mu$l d'une solution diluée au 1/60 du 1er brin d'ADNc provenant d'un extrait tissulaire ou cellulaire. Le volume final était de 6.67 $\mu$l. L'eau était utilisée comme contrôle négatif. Tous les échantillons sont gardés à 4°C pendant la préparation. Les conditions d'amplification ont été élaborées en 4 étapes: étape 1: dénaturation et activation de la polymérase par chauffage à 95°C avant amplification ; étape 2 (45 cycles): dénaturation pendant 10 sec à 95°C, hybridation pendant 10 sec à 55°C et extension pendant 6 sec à 72°C ; étape 3: incrémentation de la température de 45°C à 95°C par étape de 0.1°C ; étape 4: refroidir pendant 30 sec à 40°C pendant un cycle. Contrôles et échantillons ont été analysés en double et répétés au moins trois fois. Toutes les données de fluorescence ont été analysées avec le logiciel LightCycler 4.0 (Roche) et les résultats $C_t$ exportés comme fichiers Excel.

Une quantité importante d'ADNc de la lignée HMER-1 a été préparée avant la réalisation des PCR. Cet ADNc a été dilué et aliquoté pour servir de calibrateur pour toutes les amplifications par PCR quantitative en temps réel. Pour la quantification relative et la normalisation, la méthode comparant les $C_t$ a été utilisée (ou $E^{-\Delta\Delta C}$, avec E représentant le facteur dépendant de l'efficacité des primers au cours de la PCR). Les valeurs de $C_t$ du calibrateur et des échantillons ont été normalisées avec 3 gènes de référence dits "gènes de ménage" : PPIB (peptidyl propyl isomérase B ou cyclophiline B), $\beta$-Actin et PGK (phosphoglycérokinase).

*Immunohistochimie*

Des carottages de blocs paraffinés de tumeurs du sein ou du côlon sont insérés chacun en triple exemplaires à l'aide d'une aiguille de 600 μm (Alphelys, Plaisir, France) dans trois blocs de TMA (*Tissue Micro-Array*) pour les tumeurs du sein et six bloc de TMA pour les tumeurs du côlon. Chaque bloc est coupé en section de 4 mm d'épaisseur puis déparaffiné et réhydraté, les peroxydases endogènes sont bloquées en incubant les lames de TMA dans 5 % de peroxyde d'hydrogène dans de l'eau stérile. Un démasquage antigénique n'est pas nécessaire avant l'utilisation de l'anticorps polyclonal de lapin anti-APC11 (ab18303 pour les TMA de tumeurs du sein, ou ab115457 pour les TMA de tumeurs du côlon, Abcam, Cambridge, Royaume-Uni). Les lames sont incubées une heure à température ambiante avec l'anticorps primaire dilué au 1/200ème en utilisant une solution de dilution (Chem Mate, Daki, Trappes, France). Après rinçage en PBS, les lames sont incubées avec un anticorps secondaire biotinylé conjugué à la streptavidine peroxydase (Vectastain Elite ABC reagent Vector, Abcys, Paris, France). La révélation est réalisée par addition du substrat 3,3'-diamino benzidine (LSAB+ kit substrat pour peroxydase, DAKO, Trappes, France). Les lames sont contre-colorées en hematoxyline. Chaque lame est analysée indépendamment par l'anatomo-pathologiste et par la technicienne.

L'intensité de la coloration cytoplasmique (3 grades) ainsi que le pourcentage de cellules tumorales marquées positivement ont été déterminées. L'intensité de coloration a été notée sur une échelle de 0 à 2: "0" reflète un manque d'immunoréactivité, "1" reflète une faible immunoréactivité et "2" reflète une forte immunoréactivité. Les investigateurs n'étaient pas au courant des résultats de la RT-PCR au moment de la notation de la coloration.

Le pourcentage de cellules positives (marquage cytoplasmique) permet de discriminer les patientes présentant un risque de progression métastatique. La valeur « cut-off » est placée à 50 % de cellules positives. Les cas discordants sont revus par les deux investigateurs pour arriver à un consensus.

*Identification du phénotype épithélial et mésenchymateux*

Le phénotype épithélial et mésenchymateux a été déterminé par western blot dans les lignées cellulaires de cancer du sein et dans les modèles cellulaires humains de progression tumorale mammaire en étudiant l'expression de la E-cadhérine (marqueur épithélial) et de la vimentine (marqueur mésenchymateux).

L'analyse par Western blot a été réalisée comme décrit par Wang et al. (2003, Oncogene, 22(10) : 1486-1490). Des anticorps murins dirigés contre la E-cadhérine dilués au 1/2000ème (clone 36, Becton Dickinson, Glostrup, Denmark) et des anticorps murins dirigés contre la vimentine dilués au 1/500ème (clone V9, Dako, Trappes, France) ont été utilisés. Le phénotype épithélial et mésenchymateux a également été déterminé par l'examen de la morphologie des cellules à l'aide d'un microscope inversé (Axiovert 25, Zeiss, Oberkochen, Germany). Les lignées BT-20 et HBL-100 sont indiquées comme présentant un phénotype mixte épithélial et mésenchymateux (E-M), car les deux marqueurs testés sont positifs et qu'il n'est donc pas possible de conclure sur le phénotype E ou M de ces lignées.

*Anticorps et immunoblot*

Les culots cellulaires ont été dissociés avec du tampon RIPA (détection d'APC11) pendant 30 min dans la glace puis centrifugés 30 min à 13 000 rpm à 4°C. La concentration protéique dans le surnageant a été déterminée par la technique de Bradford à l'aide du test Biorad Protein assay (Biorad). La BSA a été utilisée comme standard. 50 μg de protéines réduites et dénaturées ont été chargés sur un gel acrylamide-bisacrylamide à 20% pour la détection d'APC11 (9,8 kDa) et la migration a été réalisée dans un tampon Tris-Gly-SDS 1X (Sigma). Le transfert a été réalisé en conditions humides avec un tampon Tris-Gly 1X (Sigma), méthanol 10 % pendant 1h. La membrane de nitrocellulose (0,2 μm) a ensuite été incubées pendant 1h avec des anticorps polyclonaux anti-APC11 (Abcam, ab18303) dilués au 1/500ème dans du TBS 1X-0,5 %-Tween-5 % lait. Après 1h d'incubation, les membranes ont été lavées 3 fois avec du TBS 1X-0,5 % Tween. Les anticorps secondaires (P0217, Dako) ont été dilués au 1/3000ème dans le même tampon et incubés pendant 1h. L'expression de la protéine APC11 a été révélée à l'aide du système Lumi-LightPLUS (Roche). L'intensité du signal a été normalisée à celui de l'actine.

La quantité relative de protéine APC11 exprimée a été estimée à l'aide du logiciel Quantity One® (Bio-Rad) qui permet d'acquérir et d'analyser des images à partir d'un équipement d'imagerie.

*Echantillons cliniques*

Les échantillons de tissu congelés sous la forme de biopsies de tissus ont été utilisés pour l'analyse ARN et des blocs de tissus inclus en paraffine ont été utilisés pour les analyses en immunohistochimie.

*Etude des ARNm*

Les échantillons de tissus ont été fournis par Eric Tabone (*Biological Resources Department,* Centre Léon Bérard, *French agreement number DC-2008-99*) et ont été collectés avant toute thérapie à partir de 106 patients souffrant de cancer du sein diagnostiqué entre 1992 and 1999 qui ont subis une intervention chirurgicale au Centre Léon Bérard (Lyon, France). Les échantillons de tissus de seins normaux ont été obtenus à partir de 4 individus sains qui ont subis une chirurgie réductrice.

*Analyse immunohistochimique*

Des blocs paraffinés de tissus fixés en formaldehyde réalisés à partir de tumeurs du sein de 111 patientes diagnostiquées au Centre Léon Bérard (*French agreement number* DC-2008-99) en 1998. Des tissus normaux de sein ont été obtenus à partir de huit prélèvements de personnes non atteintes de cancer du sein ou de personnes atteintes de cancers du sein (4 de donneuses ayant subi une chirurgie plastique et 4 provenant de tissus sains de patientes atteintes de cancers du sein). Tous les échantillons de tissus du sein ont été obtenus après l'approbation du Comité de Protection des Personnes Lyon Est, par le comité d'experts de l'institution et par le comité éthique du Centre Léon Bérard, après le consentement éclairé de chaque patiente.

Les échantillons de tissus du côlon ont été fournis par le Dr Geneviève Ranchin-Monges (Institut Paoli-Calmettes, *French agreement number DC-2008-9*) et ont été collectés avant toute thérapie à partir de 82 patients souffrant de cancer du côlon diagnostiqué entre 1990 and 1997.

*Description de la banque de données cliniques*

Les variables explicatives ont été extraites à partir de la banque de données institutionnel de cancers du sein du Centre Léon Bérard incluant toutes les patientes ayant subi une opération chirurgicale initiale au Centre Léon Bérard depuis 1996. La banque de données a été déclarée auprès des autorités de la CNIL. Les paramètres d'intérêt correspondent à: l'histoire clinique et chirurgicale de la patiente, l'histologie de la tumeur, le traitement adressé ainsi que les variables immunohistochimiques (le statut hormonal, HER2). Cette banque de données de cancers du sein est régulièrement mise à jour avec le suivi des données cliniques et autres jusqu'au décès de la patiente (lettre du médecin généraliste ou office des registres). Les informations sur l'évolution de la tumeur, avec notamment l'envahissement ganglionnaire ou les métastases à distance sont enregistrées régulièrement.

Les paramètres cliniques considérés dans cette étude s'agissant des tumeurs du sein étaient:

- les données cliniques (âge, statut ménopausique, statut métastatique au diagnostic)
- les données histologiques (type histologique, pT (taille de la tumeur), le statut des ganglions lymphatiques, le grade SBR)

- les données immunohistochimiques (statut hormonal, HER2 si disponible)
- le sous-type de cancer (les tumeurs sont classées comme suit: luminales A, luminales B, HER2+ et « basal-like » comme décrit précédemment (Creighton et al., 2009, Breast Cancer Res. Treat. 114 :287-299; Hugh et al., 2009, J. Clin. Oncol. 27 :1168-1176).

Les variables explicatives ont été extraites à partir de la banque de données institutionnelle de cancers du côlon de l'Institut Paoli-Calmettes. La banque de données a été déclarée auprès des autorités de la CNIL. Les paramètres d'intérêt correspondent à : l'histoire clinique et chirurgicale du patient, l'histologie de la tumeur, le traitement adressé.

Les paramètres cliniques considérés dans cette étude s'agissant des tumeurs du côlon, étaient:

- les données cliniques (âge, sexe, stade (1 à 4, voir détails ci-dessous), statut métastatique au diagnostic, récidive (récidive locale (maladie résiduelle) et récidive à distance (métastases)) ;

  • Stade 0 : la tumeur est *in situ,* ce qui signifie qu'elle est très superficielle et qu'elle n'envahit pas la sous-muqueuse, que les ganglions lymphatiques ne sont pas atteints et qu'il n'y a pas de métastase à distance.
  • Stade I : la tumeur envahit la deuxième couche (sous-muqueuse) ou la couche musculaire (musculeuse) de la paroi du côlon ou du rectum, les ganglions lymphatiques ne sont pas atteints et il n'y a pas de métastase à distance.
  • Stade II : les cellules cancéreuses ont traversé plusieurs couches de la paroi du côlon ou du rectum, mais aucun ganglion n'est atteint et il n'y a pas de métastase à distance.
  • Stade III : les cellules cancéreuses ont envahi les ganglions lymphatiques proches de la tumeur.

• Stade IV : le cancer s'est propagé au-delà du côlon ou du rectum, vers des emplacements ou des organes éloignés, généralement le foie ou les poumons.

- les données histologiques (type histologique, le statut des ganglions lymphatiques).

*Analyses statistiques*

*Expression en ARNm et résultats d'immunohistochimie (TMA)*

Une transformation de $\log_2$ a été appliquée à l'expression de l'ARNm d'APC11 à partir du moment où les variables ne rencontrent pas la normalité. Basés sur les résultats en test Kolmogorov-Smirnov, la significativité statistique pour une répartition par rapport à la normalité était p=0.002 et p=0.291 avant et après transformation respectivement. Basé sur l'expérimentation, les Q-PCRs ont été emboîtées avec les RTs, elles-mêmes emboîtées avec les extractions, elles-mêmes emboîtées avec les lignées cellulaires suggérant l'utilisation d'un modèle hiérarchique linéaire (Sullivan et al., 1999, Stat. Med. 18 :855-888 ; Goldstein et al., 2003, Multilevel Statistical models. Arnold Publishers, London). Par conséquent, la moyenne « $\log_2$ mean » de l'expression de l'ARNm d'APC11 pour les lignées de cancer du sein était estimée en utilisant le modèle mixte linéaire à 4 niveaux suivant (LMM):

$$\log_2 APC/C_{irel} = \mu + \alpha_l + \delta_{el} + \gamma_{rel} + \varepsilon_{irel}$$

où $\mu$ était la moyenne de toutes les valeurs de l'expression de l'ARNm d'APC11 après transformation $\log_2$ pour les effets randomisés, $\alpha_l$ représentait la variation entre cellules, $\delta_{el}$ la variation entre extraction, $\gamma_{rel}$ variation entre RTs ou à l'intérieur d'une extraction et $\varepsilon_{irel}$ la variation intra RT ou la mesure de l'erreur. Les effets aléatoires étaient assurés d'être indépendants et normalement distribués entre la valeur de zéro et le coefficient de variance comme suit:

$$\alpha_l \sim N(0,\sigma_\alpha^2)\, , \; \delta_{el} \sim N(0,\sigma_\delta^2)\, , \; \gamma_{rel} \sim N(0,\sigma_\gamma^2) \; \text{et} \; \varepsilon_{irel} \sim N(0,\sigma_\varepsilon^2)\, .$$

La valeur $2\mu$ a été reportée avec son intervalle de confiance de 95% (95% IC) au niveau des résultats et représente la moyenne de l'expression en ARNm de APC11. Des estimations ont été réalisées en utilisant la méthode de maximum de vraisemblance à partir de la bibliothèque de nlme du logiciel R *([*R Development Core Team (2007). R: A language and environment for statistical computing. R Foundation for Statistical Computing, Vienna, Austria. ISBN 3-900051-07-0, URL http://www.R-project.org]). Pour les tests de ratios de vraisemblance, le niveau de significativité statistique était de 0.05.

Une comparaison de la distribution des paramètres cliniques selon le pourcentage des cellules marquées montrant un marquage cytoplasmique ($\leq$50% versus >50%) a été réalisée à l'aide du test de Pearson's $\chi^2$ test; le test de Fisher exact a été utilisé quand le nombre de patients était faible. Le test de Wilcoxon a été utilisé pour les variables quantitatives.

*Analyses de survie*

Les analyses de survie pertinentes dans cette étude ont été des études de survie sans progression métastatique (SPM) définies comme le temps entre la survenue d'un évènement métastatique et le diagnostic initial de cancer ou le suivi des patients sans métastases (observations recensées). Les analyses de survie ont été réalisées parmi un sous-groupe de patientes atteintes de cancer du sein sans métastases au diagnostic. Les estimations de survie ont été calculées à l'aide de la méthode de Kaplan-Meier (Kaplan and Meier, 1958, J. Am. Stat. Assoc. 53 ;457-481). La différence dans l'estimation de survie entre les groupes présentant des quantités relatives de protéine APC11 $\leq$50% versus >50% a été estimée par le test de log-rank (Peto et al., 1977, Br. J., Cancer, 35 :1-39) et les « hazard ratios » ont été calculées à l'aide du modèle de Cox.

Les analyses statistiques ont été réalisées avec le logiciel SAS version 9.1 [*SAS OnlineDoc®, Version 9, Copyright © 1999 by SAS Institute Inc., Cary, NC, USA*] et R [R Development Core Team (2007). R: A language and environment for statistical computing. R Foundation for Statistical Computing, Vienna, Austria. ISBN 3-900051-07-0, URL http://www.R-project.org] à l'aide du pack "nlme" [*Jose Pinheiro, Douglas Bates, Saikat DebRoy and Deepayan Sarkar and the R Core team. (2007). nlme: Linear and Nonlinear Mixed Effects Models. R package version 3.1-89*].

## Expression de APC11 dans des lignées cellulaires de cancer du colon

L'expression des ARNm et de la protéine APC11 ont été étudiées par RT-PCR quantitative en temps réel et Western Blot respectivement dans des lignées cellulaires de cancers du côlon, comme indiqué dans la section « Matériels et Méthodes » ci-dessus. Les ARNm ont été obtenus à partir de 15 lignées cellulaires de l'ATCC établies à partir de cancers colorectaux (Caco2, Co-115, Colo-320, FET, HCT-116, HCT-15, HT-29, Lovo, LS-1034, LS-174T, SW-1116, SW-48, SW-480, SW-620, SW-837), de la lignée colorectale EB et des lignées IS1 (Isreco1), IS2, IS3, TC7 et TC71. Les protéines ont été obtenues à partir de 6 lignées cellulaires de l'ATCC établies de cancer du côlon (HCT-15, HT-29, Lovo, SW-480, SW-620, SW-1116), des cellules Co-115, Isreco (IS2), TC-7 et TC-71, et des cellules HME-1.

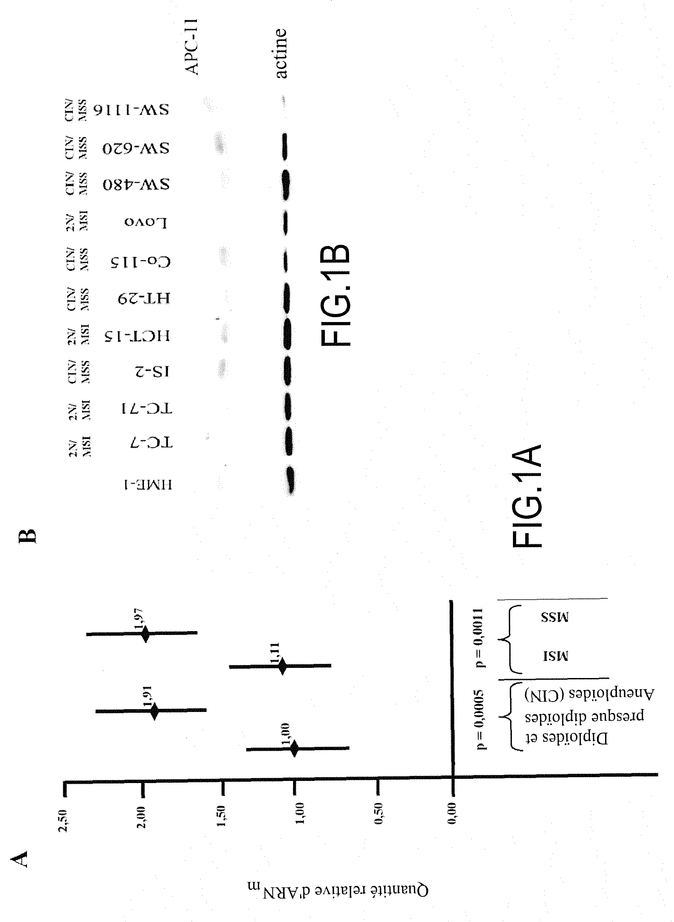

Les résultats sont regroupés sur la figure 1.

Concernant les ARNm, les résultats montrent une augmentation significative du niveau d'expression de APC11 dans les lignées cellulaires aneuploïdes, c'est-à-dire associées à une instabilité chromosomique (CIN), comparé aux lignées cellulaires diploïdes ou « presque diploïdes » (2N) (CIN:IC moyen = 1.906 (1.595-2.278) vs diplôïde : IC moyen = 0.995 (0.741-1.337), p = 0.0005). Les lignées cellulaires à instabilité microsatellite (MSI *MicroSattelite Instability)* présentent un niveau d'ARNm inférieur à celui des lignées de tumeurs coliques à stabilité microsatellitaire (souvent instables au niveau chromosomique (MSI : IC moyen = 1.11 (0.852-1.437) vs MSS (*MicroSattelite Stability*) : IC moyen = 1.97 (1.635-2.362). De plus, des niveaux d'ARNm supérieurs ont été mesurés dans les cellules cancéreuses par rapport à ceux mesurés dans des cellules normales (p=0.0025)(résultats non représentés)(ces résultats ont-ils été obtenus à partir des lignées cellulaires décrites.

Quatre des six lignées cellulaires aneuploïdes surexpriment la protéine APC11 et trois quart des lignées cellulaires diploïdes (2N) présentent un niveau d'expression de la protéine APC11 inférieur à celui mesuré dans la lignée cellulaire HME-1 (lignée cellulaire épithéliale mammaire immortalisée non tumorale).

Des résultats similaires ont été obtenus avec d'autres lignées cellulaires de cancer du côlon. Les mesures semi-quantitatives du niveau d'expression de la protéine APC11 montrent une concordance de 60 % avec les niveaux d'ARNm. Au vu de ces résultats, APC11 peut donc être considéré comme un marqueur d'agressivité tumorale.

## Expression de APC11 dans des lignées cellulaires de cancer du sein

L'expression des ARNm et de la protéine APC11 ont été étudiées par RT-PCT et Western Blot respectivement dans des lignées cellulaires de cancers du sein, comme indiqué dans la section « Matériels et Méthodes » ci-dessus. Les ARNm ont été obtenus à partir de 21 lignées cellulaires de l'ATCC établies à partir de cancers du sein (BT20, BT474, Cal-51, Cama1, HCC 1937, Hs578T, MCF7, MDA-MB-157, MDA-MB-231, MDA-MB-361, MDA-MB-436, MDA-MB-453, MDA-MB-459, MDA-MB-549, SKBr3, T47D, UACC-812, ZR-75-1, HBL-100, MCF-10A, MCF12A). Les protéines ont été obtenues à partir de 8 lignées cellulaires de l'ATCC établies de cancers du sein (MCF-7, T47D, BT-20, UACC-812, MM-157, MM-231, MM-549 et HLB-100) et des cellules HME-1.

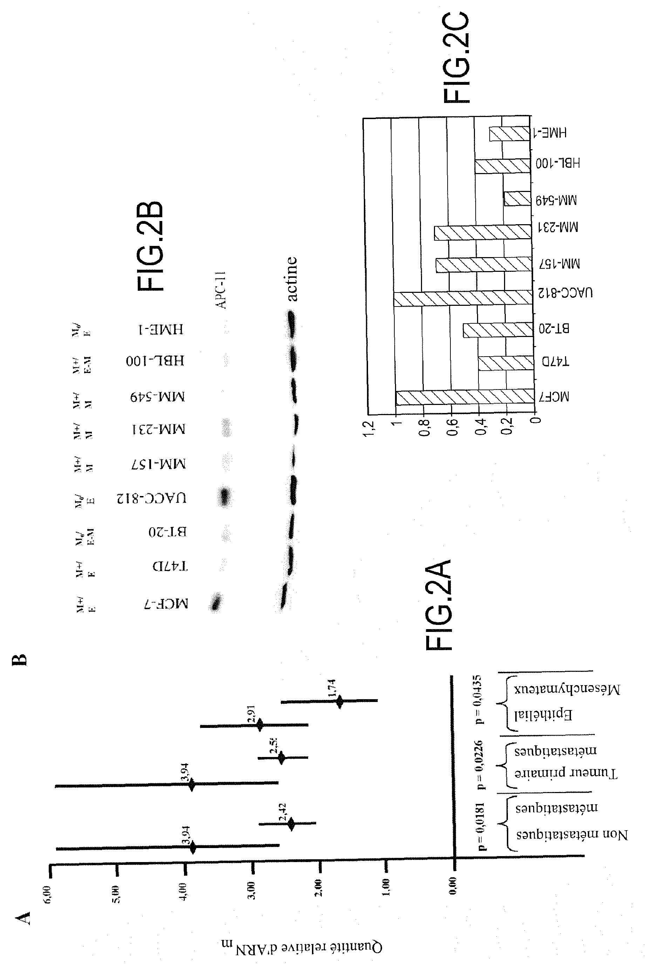

Les résultats sont regroupés sur la figure 2.

Concernant les ARNm, les résultats montrent que l'expression d'APC11 est significativement inférieure dans les lignées cellulaires métastatiques par rapport à celui présent dans les lignées non métastatiques (non métastatiques : IC moyen = 3.942 (2.611-5.953) vs métastatiques : IC moyen = 2.423 (2.046-2.87); p = 0.0181).

Une diminution significative a également été observée dans les lignées d'origine métastatiques par rapport aux lignées cellulaires issues de tumeurs primaires (tumeurs primaires : IC moyen = 3.942 ((2.611-5.953) vs métastatiques : IC moyen = 2.551 (2.201-2.957); p = 0.0226).

De plus, le niveau d'ARNm est significativement inférieur dans les lignées de phénotype mésenchymateux par rapport à celui mesurée dans les lignées cellulaires de phénotype épithélial (épithélial: IC moyen = 2.908 ((2.210-3.827) vs phénotype mésenchymateux: IC moyen = 1.745 (1.172-2.598); p = 0.0435) ; le phénotype mésenchymateux étant généralement corrélé à une susceptibilité métastatique accrue.

La quantité de protéine APC11 présente dans la lignée de cancer du sein UACC-812 est prise comme valeur de référence car elle est non métastatique et de phénotype épithélial (valeur arbitraire de 1). De façon similaire aux résultats obtenus pour l'ARNm, ces résultats montrent que l'expression de la protéine APC11 est plus faible dans les lignées de phénotype mésenchymateux par rapport à l'expression quantifiée dans les lignées de phénotype épithélial. Les informations cliniques sur les lignées de l'ATCC ne permettent pas de savoir si les lignées de cancers du sein ont été établies à partir de tumeurs métastatiques au diagnostic, ou si les patientes ont présenté une rechute métastatique au cours du temps.

Des résultats similaires ont été obtenus avec d'autres lignées cellulaires de cancer du sein. Les mesures semi-quantitatives du niveau d'expression de la protéine APC11 montrent une concordance de 60 % avec les niveaux d'ARNm.

**Expression de APC11 dans des tumeurs primaires de cancer du sein**

L'expression de APC11 a été étudiée sur des biopsies de tumeurs primaires du sein par la mesure des ARNm.

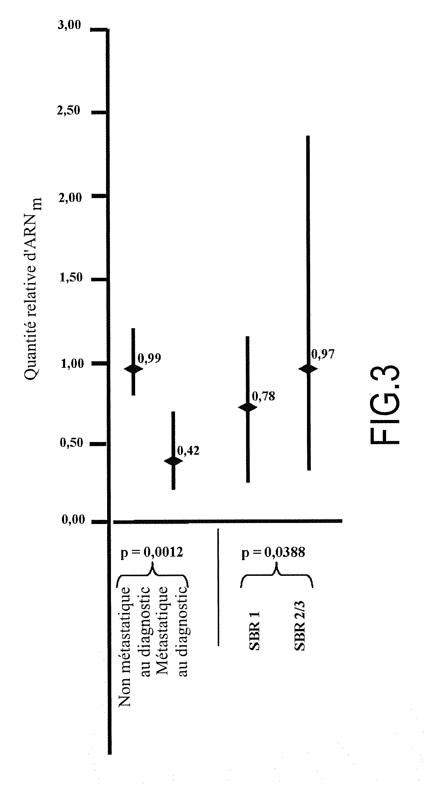

Les résultats sont regroupés sur la figure 3.

Les résultats montrent une diminution significative du niveau d'ARNm de APC11 dans les tumeurs métastatiques au diagnostic (non métastatiques au diagnostic : IC moyen = 0.99 (0.808-1.217) vs métastatiques au diagnostic : IC moyen = 0.42 (0.268-0.669); p = 0.0012).

Dans les échantillons de tumeur primaire de cancer du sein, l'expression de APC11 est donc corrélée à un paramètre clinique, c'est-à-dire qu'il existe une association claire entre l'expression de APC11 en ARNm et la présence de métastases au diagnostic.

**Pertinence pronostic et clinique de l'expression de la protéine APC11 dans des tumeurs primaires de cancer du sein**

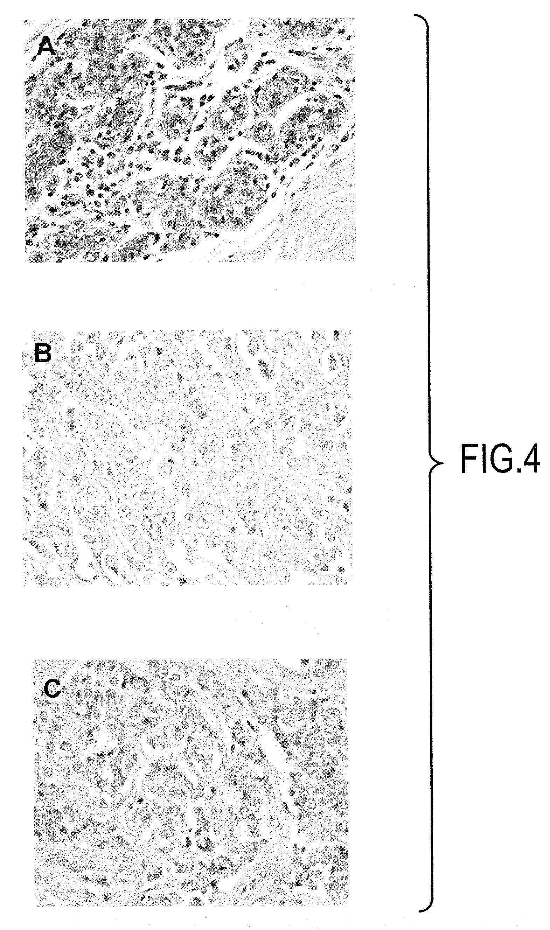

L'expression de la protéine APC11 a été étudiée par analyse TMA (*Tissue MicroArray).* Une analyse préliminaire par immunohistochimie a été réalisée avec 8 tissus de glande mammaire normale et a montré que l'expression de la protéine APC11 est non uniforme dans le cytoplasme des cellules luminales. Le signal dans le cytoplasme des cellules luminales est facilement détectable (correspondant à une intensité de 2 et 50 % de cellules marquées). Aucune immunoréactivité positive n'est détectée dans les cellules basales ou les cellules stromales (figure 4A).

Dans les cellules de tumeurs mammaires, le profil d'expression de la protéine APC11 est très variable. Certaines tumeurs sont négatives pour la coloration APC11 (intensité 0) (figure 4B), d'autres tumeurs présentent une intensité de coloration plus faible que celle trouvée dans des tissus normaux (intensité 1 et $\leq$ 50 % de cellules marquées) alors que d'autres tumeurs présentent une intensité de coloration comparable à celle trouvée dans des tissus normaux (intensité 2 et > 50 % de cellules marquées) (figure 4C).

Les caractéristiques cliniques de la population de tumeurs analysée en TMA sont décrites dans le **Tableau 1** ci-dessous. Dans cette population de tumeurs, 3 patients sur 111 présentent des métastases à distance au moment du diagnostic (contre 21 sur 106 dans la population ARNm).

**Tableau 1**

| Comparaison des populations tumorales analysées en TMA et en ARNm | | | | |
|---|---|---|---|---|
| m Mean $\pm$ SD (median [min,max] (%) | population TMA N=111 | | population ARNm N=106 | |
| Age au diagnostic (années) | | | | |
| N | 111 | | 96 | |
| Mean (std) | 58.55 (12.73) | | 57.36 (12.92) | |
| Median (min - max) | 56.70 (37.60 - 87.40) | | 55.40 (32.00 - 84.40) | |
| Q1 - Q3 | 48.20 - 69.90 | | 48.25 - 66.40 | |
| Statut Menopause: | | | | |
| - | | | 14 | |
| Non | 37 | (33.3%) | 32 | (34.8%) |
| Oui | 74 | (66.7%) | 60 | (65.2%) |
| statut métastatique: | | | | |
| - | | | 1 | |
| M0 | 108 | (97.3%) | 84 | (80.0%) |
| M1 | 3 | (2.7%) | 21 | (20.0%) |
| Taille de la tumeur (mm) | | | | |
| - | | | 3 | |
| < 30 mm | 69 | (62.2%) | 35 | (34.0%) |
| > = 30 mm | 42 | (37.8%) | 68 | (66.0%) |
| Type histologique : | | | | |
| Lobulaire | 8 | (7.2%) | 7 | (6.6%) |
| canalaire | 94 | (84.7%) | 91 | (85.8%) |
| Mixte | | | 1 | (0.9%) |

(suite)

| Comparaison des populations tumorales analysées en TMA et en ARNm | | | | |
|---|---|---|---|---|
| m Mean ± SD (median [min,max] (%) | population TMA N=111 | | population ARNm N=106 | |
| Autres | 9 | (8.1%) | 7 | (6.6%) |
| Grade histologique (SBR): | | | | |
| - | | | 8 | |
| 1 | 20 | (18.0%) | 12 | (12.2%) |
| 2/3 | 91 | (82.0%) | 86 | (87.8%) |
| Statut ganglionnaire : | | | | |
| N0 | 46 | (41.4%) | 19 | (17.9%) |
| Micro metastases | 18 | (16.2%) | 7 | (6.6%) |
| Macro metastases | 47 | (42.3%) | 80 | (75.5%) |
| Récepteurs aux oestrogènes : % cellules marquées (comptage): | | | | |
| - | | | 33 | |
| ER- (< 10%) | 12 | (10.8%) | 14 | (19.2%) |
| ER+ (≥ 10%) | 99 | (89.2%) | 59 | (80.8%) |
| Recepteurs à la progestérone : % cellules marquées (comptage) | | | | |
| - | | | 33 | |
| PR- (< 10%) | 24 | (21.6%) | 22 | (30.1%) |
| PR + (≥ 10%) | 87 | (78.4%) | 51 | (69.9%) |
| Statut HER2: | | | | |
| - | 2 | | 30 | |
| 0/+ | 99 | (90.8%) | 63 | (82.9%) |
| ++/+++ | 10 | (9.2%) | 13 | (17.1%) |
| Sous-type tumoral luminal A | | | | |
| - | | | 27 | |
| Non | 43 | (38.7%) | 55 | (69.6%) |
| Oui | 68 | (61.3%) | 24 | (30.4%) |
| Sous-type tumoral luminal B | | | | |
| - | | | 4 | |
| Non | 80 | (72.1%) | 70 | (68.6%) |
| Oui | 31 | (27.9%) | 32 | (31.4%) |
| Sous-type tumoral | | | | |
| - | | | 44 | |
| Luminal A | 68 | (61.3%) | 24 | (38.7%) |
| Luminal B | 31 | (27.9%) | 32 | (51.6%) |
| HER2+ | 2 | (1.8%) | 0 | |
| Basal-like | 10 | (9.0%) | 6 | (9.7%) |

45 tumeurs sur 111 (40,5 %) ont présenté ≤ 50 % de cellules marquées et 66 sur 111 (59,5 %) ont présenté plus de 50 % de cellules marquées **(Tableau 2).**

**Tableau 2**

| Mean ± SD (median [min,max] (%) | % cellules marquées APC11 | | Test |
|---|---|---|---|
| | <=50 N=45 | >50 N=66 | |
| **Age au diagnostic (années)** | | | Wilcoxon |
| N | 45 | 66 | **P = 0.026** |
| Mean (std) | 55.34 (12.07) | 60.73 (12.79) | |
| Median (min - max) | 53.00 (37.90 - 82.70) | 59.25 (37.60 - 87.40) | |
| Q1 - Q3 | 46.40 - 63.00 | 50.00 - 72.30 | |
| **Statut ménopause :** | | | Fisher Exact |
| Non | 18 (40.0%) | 19 (28.8%) | P = 0.227 |
| Oui | 27 (60.0%) | 47 (71.2%) | Fisher |
| **Statut métastatique :** | | | Exact |
| M0 | 45 (100.0%) | 63 (95.5%) | P = 0.270 |
| M1 | 0 (0.0%) | 3 (4.5%) | |
| **Taille de la tumeur (mm)** | | | Fisher Exact |
| < 30 mm | 21 (46.7%) | 48 (72.7%) | **P = 0.009** |
| > = 30 mm | 24 (53.3%) | 18 (27.3%) | |
| **Type histologique:** | | | Fisher Exact |
| Lobulaire | 2 (4.4%) | 6 (9.1%) | P = 0.724 |
| Canalaire | 39 (86.7%) | 55 (83.3%) | |
| Autres | 4 (8.9%) | 5 (7.6%) | |
| **Grade histologique (SBR):** | | | Fisher Exact |
| 1 | 6 (13.3%) | 14 (21.2%) | P = 0.326 |
| 2/3 | 39 (86.7%) | 52 (78.8%) | |
| **Staut ganglionnaire:** | | | Fisher Exact |
| N0 | 14 (31.1%) | 32 (48.5%) | **P = 0.072** |
| Micro metastases | 6 (13.3%) | 12 (18.2%) | |
| Macro metastases | 25 (55.6%) | 22 (33.3%) | |
| **Récepteurs aux oestrogènes: % cellules marquées (comptage):** | | | Fisher Exact P = 0.221 |
| ER- (< 10%) | 7 (15.6%) | 5 (7.6%) | |
| ER+ (≥ 10%) | 38 (84.4%) | 61 (92.4%) | |
| **Récepteurs aux oestrogènes: % cellules marquées (comptage)** | | | Fisher Exact P = 0.640 |
| PR- (< 10%) | 11 (24.4%) | 13 (19.7%) | |
| PR+ (≥ 10%) | 34 (75.6%) | 53 (80.3%) | |
| **HER2 status** | | | Fisher Exact |
| - | 1 | 1 | P = 0.086 |
| + | 37 (84.1%) | 62 (95.4%) | |
| +++ | 7 (15.9%) | 3 (4.6%) | |
| **Sous-type tumoral Luminal A** | | | Fisher Exact |
| Non | 22 (48.9%) | 21 (31.8%) | P = 0.078 |
| Oui | 23 (51.1%) | 45 (68.2%) | |
| **Sous-type tumoral Luminal B** | | | Fisher Exact |
| Non | 30 (66.7%) | 50 (75.8%) | P = 0.389 |
| Oui | 15 (33.3%) | 16 (24.2%) | |

(suite)

| Mean ± SD (median [min,max] (%) | % cellules marquées APC11 | | | | Test |
|---|---|---|---|---|---|
| | <=50 N=45 | | >50 N=66 | | |
| Sous-type tumoral | | | | | Fisher Exact P = 0.256 |
| Luminal A | 23 | (51.1%) | 45 | (68.2%) | |
| Luminal B | 15 | (33.3%) | 16 | (24.2%) | |
| HER2+ | 1 | (2.2%) | 1 | (1.5%) | |
| Basal-like | 6 | (13.3%) | 4 | (6.1%) | |

De plus, les tumeurs qui ont présenté plus de 50 % de cellules marquées étaient plus fréquemment des tumeurs de petite taille (< 30 mm 72,7% vs ≥ 30 mm 27,3%, p = 0.009, Tableau 2) et les tumeurs qui ont présenté ≤ 50 % de cellules marquées sont significativement associées à la présence de macrométastases (macrométastases 55,6 % vs N0/micrométastases 44,4 %, p = 0.072, Tableau 2). Enfin, une association significative entre les tumeurs qui ont présenté un pourcentage élevé de cellules marquées a également été mise en évidence pour le sous-type luminal A de cancers (luminal A 68,2 % vs non luminal A 31,8 %, p = 0.078, Tableau 2).

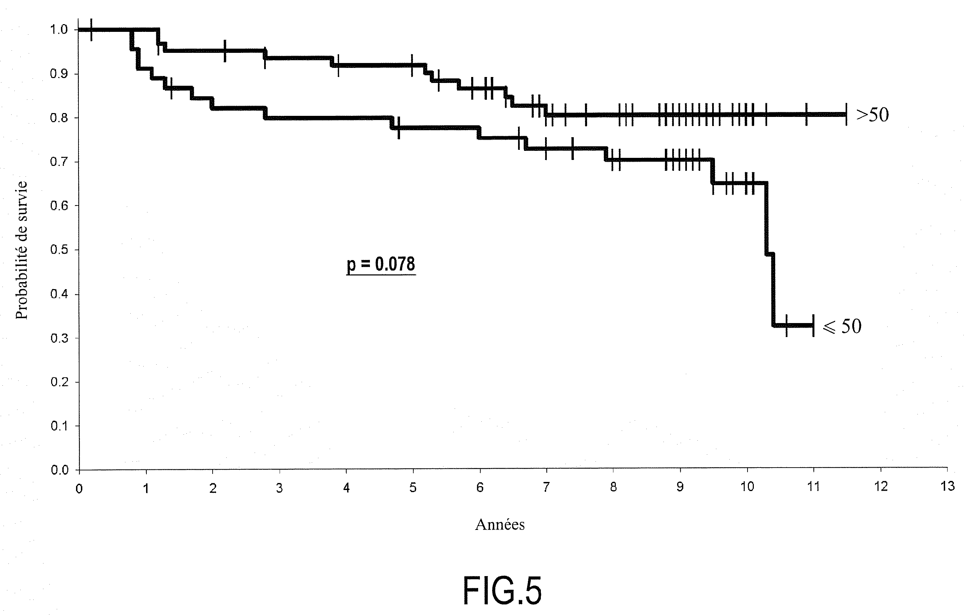

La pertinence de l'utilisation de l'expression de la protéine APC11 en tant que marqueur pronostic chez les patients atteints de cancer du sein a ensuite été étudiée. Ainsi, il a été mis en évidence que le niveau d'expression de la protéine APC11 est pertinent puisque la population de patients qui ont ≤ 50 % de cellules marquées pour APC11 présentent une survie sans métastases significativement réduite (p = 0,078, figure 5). Par conséquent, les patientes dont les tumeurs présentent moins de 50 % de cellules marquées positivement pour APC11 présentent un risque accru de développer des métastases au cours du temps.

**Pertinence pronostic et clinique de l'expression de la protéine APC11 dans des tumeurs primaires de cancer colorectal (ou cancer du côlon)**

L'expression de la protéine APC11 a été étudiée par analyse TMA (*Tissue MicroArray*). Une analyse préliminaire par immunohistochimie a été réalisée sur du tissu sain de colon et a montré que l'expression de la protéine APC11 est détectée dans le cytoplasme. Le signal dans le cytoplasme est facilement détectable.

Dans les cellules de tumeurs colorectales, le profil d'expression de la protéine APC11 est très variable. Certaines tumeurs sont négatives pour la coloration APC11 (intensité 0), d'autres tumeurs présentent une intensité de coloration comparable à celle trouvée dans des tissus normaux (intensité 1 et ≤50 % de cellules marquées) alors que d'autres tumeurs présentent une intensité de coloration plus forte que celle trouvée dans des tissus normaux (intensité 2 et > 50 % de cellules marquées).

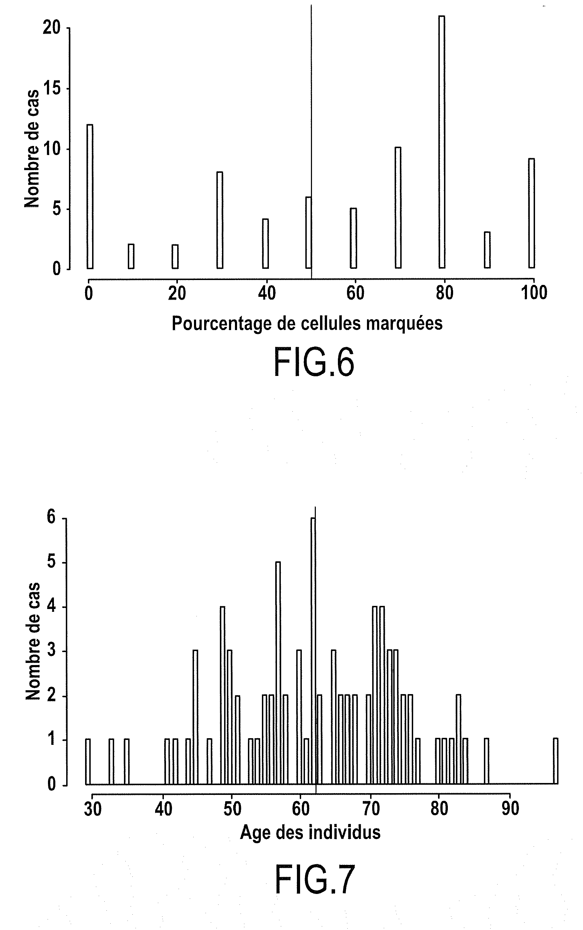

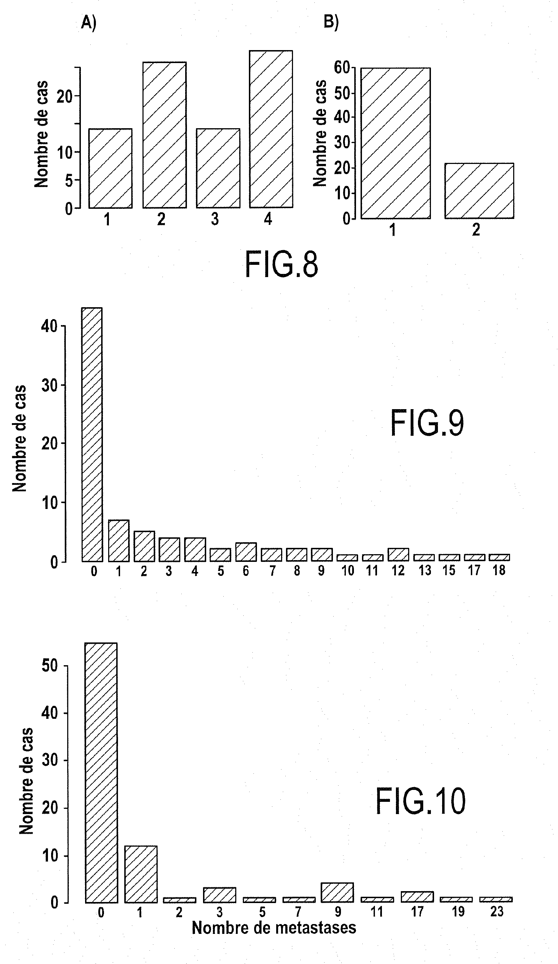

Les caractéristiques cliniques de la population de tumeurs analysée en TMA sont décrites dans les figures 6 à 10.

La pertinence de l'utilisation de l'expression de la protéine APC11 en tant que marqueur pronostic chez les patients atteints de cancer du côlon a ensuite été étudiée. Il n'y a pas d'association significative entre le pourcentage de cellules marquées et les stades tumoraux 1, 2 et 3. Par contre, les tumeurs qui ont présenté plus de 50 % de cellules marquées étaient plus fréquemment des tumeurs de stade 4 (78,5% des tumeurs de stade 4 présentent plus de 50% de cellules marquées, tableau 3).

**Tableau 3**

| Cellules marquées | Stade | | | |
|---|---|---|---|---|
| | 1 | 2 | 3 | 4 |
| ≤ 50% | 9 | 12 | 7 | 6 |
| > 50% | 5 | 14 | 7 | 22 |

De plus, les tumeurs qui ont présenté plus de 50 % de cellules marquées sont significativement associées à la présence de métastases (77,7% des tumeurs métastatiques présentent plus de 50% de cellules marquées, Tableau 4).

**Tableau 4**

| Cellules marquées | Métastase(s) | |
| --- | --- | --- |
| | Non | Oui |
| ≤ 50% | 28 | 6 |
| > 50% | 27 | 21 |

De façon très intéressante, les tumeurs provenant de patients avec récidive sont retrouvées de façon plus importante dans le groupe présentant plus de 50 % de cellules marquées (86,3 % des patients avec une récidive présentent plus de 50% de cellules marquées, Tableau 5).

**Tableau 5**

| Cellules marquées | Maladie résiduelle | |
| --- | --- | --- |
| | Non | Oui |
| ≤ 50% | 31 | 3 |
| > 50% | 29 | 19 |

Deux études statistiques ont été utilisées, une analyse binarisée a été réalisée avec une valeur « cut-off » à 50% de cellules marquées (≤50% et >50%), avec le test Pearson's $\chi^2$, l'association avec le stade tumoral montre une valeur p = 0.0404, l'association avec la présence de métastases montre une valeur p = 0.0251 et l'association avec la récidive montre une valeur p = 0.004. L'autre analyse linéaire montre une association significative avec le stade (p= 0.0296) et une association significative avec la récidive (p = 0.0062).

Ainsi, il a été mis en évidence que le niveau d'expression de la protéine APC11 est pertinent. Par conséquent, les patients atteints de tumeurs colorectales dont les tumeurs présentent plus de 50 % de cellules marquées positivement pour APC11 présentent un risque accru de récidive, à la fois de récidive locale mais également de récidive « à distance » c'est-à-dire de développer des métastases.

L'ensemble de ces résultats montre que l'expression de la protéine APC11 est un marqueur d'agressivité et/ou de risque de métastases chez les patients atteints de cancer du sein ou du colon, mais de manière opposée.

L'invention n'est pas limitée aux exemples décrits et représentés car diverses modifications peuvent y être apportées sans sortir de son cadre.

## Revendications

1. - Procédé d'identification d'un cancer agressif ou susceptible de développer des métastases chez un patient atteint de cancer du sein ou du côlon comprenant les étapes consistant à :

   - mesurer le niveau d'expression de la protéine APC11 dans un échantillon issu dudit patient, et
   - classer le cancer comme agressif ou susceptible de développer des métastases lorsqu'une différence significative du niveau d'expression de la protéine APC11 est mise en évidence par rapport à une valeur de référence, ladite valeur de référence pouvant être :

     i) une valeur seuil d'expression, obtenue en déterminant le nombre ou la proportion de cellules qui expriment la protéine APC11 ou encore la quantité ou l'intensité d'expression de la protéine APC11 au sein d'échantillons issus de patients atteints de cancer du sein ou du côlon et dont on connait le sort ultérieur s'agissant de l'agressivité du cancer, et/ou du développement de métastases,
     ii) un niveau d'expression de la protéine APC11 mesurée dans un échantillon biologique issu d'un sujet sain ou bien atteint du même type de cancer mais qui n'est pas agressif ni susceptible de métastaser,
     iii) une valeur moyenne du niveau d'expression de la protéine APC11 qui est détecté sur un pool d'échantillons biologiques issus de sujets sains ou de sujets atteints du même type de cancer mais qui n'est pas agressif ni susceptible de métastaser.

2. - Procédé selon la revendication 1, **caractérisé en ce que** ledit patient est atteint de cancer du sein et **en ce que** le cancer est classé comme susceptible de développer des métastases lorsqu'une diminution significative du niveau d'expression de APC11 est mise en évidence dans l'échantillon issu dudit patient par rapport à la valeur de référence.

3. - Procédé selon la revendication 2, **caractérisé en ce que** la diminution est d'au moins 50 % de l'expression de APC11.

4. - Procédé selon l'une quelconque des revendications 1 à 3, **caractérisé en ce que** ledit patient est atteint d'un cancer du sein qui avait été considéré comme de bon pronostic au moment du diagnostic.

5. - Procédé selon la revendication 1, **caractérisé en ce que** ledit patient est atteint d'un cancer du côlon et que le cancer est classé comme agressif lorsqu'une augmentation significative du niveau d'expression de APC11 est mise en évidence dans l'échantillon issu dudit patient à tester par rapport à la valeur de référence.

6. - Procédé selon la revendication 5, **caractérisé en ce que** le cancer est classé comme agressif lorsque l'augmentation est d'au moins 50 % de l'expression de APC11.

**7.** - Procédé selon l'une des revendications 5 et 6, **caractérisé en ce que** ledit patient est atteint d'un cancer du côlon sans envahissement ganglionnaire.

**8.** - Procédé selon l'une quelconque des revendications précédentes, **caractérisé en ce que** ladite protéine APC11 est la protéine APC11 humaine.

**9.** - Procédé selon l'une quelconque des revendications précédentes, **caractérisé en ce que** l'échantillon issu dudit patient est un tissu, de préférence issu de la tumeur primaire.

**10.** - Procédé selon l'une quelconque des revendications précédentes, **caractérisé en ce que** la valeur de référence correspond au niveau d'expression de la protéine APC11 mesurée dans un échantillon issu d'un sujet sain qui n'est pas atteint de cancer.

**11.** - Procédé selon l'une quelconque des revendications précédentes, **caractérisé en ce que** le niveau d'expression de la protéine APC11 est quantifié en utilisant des anticorps anti-APC11.

**12.** - Procédé selon la revendication 11, **caractérisé en ce que** la quantification du niveau d'expression de la protéine APC11 est déterminée par immunohistochimie.

**13.** - Utilisation de la mesure du niveau d'expression d'APC11 in vitro en tant que biomarqueur pronostic des cancers du sein et du côlon.

**14.** - Utilisation selon la revendication 13, **caractérisée en ce que** le pronostic consiste à déterminer l'agressivité ou la susceptibilité de développer des métastases desdits cancers.

**15.** - Utilisation selon l'une des revendications 13 et 14, **caractérisée en ce que** le pronostic consiste à déterminer la susceptibilité de développer des métastases du cancer du sein.

**16.** - Utilisation selon l'une des revendications 13 et 14, **caractérisée en ce que** le pronostic consiste à déterminer le risque de récidive des cancers du côlon, et en particulier de récidive locale.


**Patentansprüche**

**1.** Verfahren zur Identifizierung von aggressivem oder wahrscheinlich Metastasen entwickelndem Krebs bei einem Patienten mit Dickdarmkrebs, umfassend die Schritte, die darin bestehen:

- das Expressionsniveau des Proteins APC11 in einer dem Patienten entnommenen Probe zu messen, und
- den Krebs als aggressiv oder wahrscheinlich Metastasen entwickelnd einzustufen, wenn ein signifikanter Unterschied des Expressionsniveaus des Proteins APC11 im Vergleich mit einem Referenzwert aufgezeigt wird, wobei der Referenzwert sein kann:

i) ein Expressionsgrenzwert, der erhalten wird, wobei die Anzahl oder das Verhältnis von Zellen, die das Protein APC11 oder auch die Menge oder die Intensität der Expression des Proteins APC11 innerhalb von Proben, die von Patienten mit Brust- oder Dickdarmkrebs stammen, exprimieren, bestimmt werden, deren weiteres Schicksal im Hinblick auf die Aggressivität des Krebses und/oder die Entwicklung von Metastasen bekannt ist,
ii) ein Expressionsniveau des Proteins APC11, gemessen in einer biologischen Probe, die von einer gesunden Person oder einer Person stammt, die an demselben Krebstyp leidet, der aber nicht aggressiv ist oder wahrscheinlich Metastasen entwickelt,
iii) ein Durchschnittswert des Expressionsniveaus des Proteins APC11, das an einem Pool von biologischen Proben entdeckt wird, die von gesunden Personen oder von Personen stammen, die an demselben Krebstyp leiden, der aber nicht aggressiv ist oder wahrscheinlich Metastasen entwickelt.

**2.** Verfahren nach Anspruch 1, **dadurch gekennzeichnet, dass** der Patient an Brustkrebs leidet, und dass der Krebs als wahrscheinlich Metastasen entwickelnd eingestuft wird, wenn eine signifikante Verringerung des Expressionsniveaus von APC11 in der Probe, die von dem Patienten stammt, im Vergleich mit dem Referenzwert entdeckt wird.

3. Verfahren nach Anspruch 2, **dadurch gekennzeichnet, dass** die Verringerung mindestens 50 % der Expression von APC11 ausmacht.

4. Verfahren nach einem der Ansprüche 1 bis 3, **dadurch gekennzeichnet, dass** der Patient an Brustkrebs leidet, der zum Zeitpunkt der Diagnose als mit einer guten Prognose betrachtet wurde.

5. Verfahren nach Anspruch 1, **dadurch gekennzeichnet, dass** der Patient an Dickdarmkrebs leidet, und dass der Krebs als aggressiv eingestuft wird, wenn eine signifikante Erhöhung des Expressionsniveaus von APC11 in der Probe, die von dem zu testenden Patienten stammt, im Vergleich mit dem Referenzwert entdeckt wird.

6. Verfahren nach einem der Ansprüche 5, **dadurch gekennzeichnet, dass** der Krebs als aggressiv eingestuft wird, wenn die Erhöhung mindestens 50 % der Expression von APC11 ausmacht.

7. Verfahren nach einem der Ansprüche 5 und 6, **dadurch gekennzeichnet, dass** der Patient an Dickdarmkrebs ohne Lymphknotenbefall leidet.

8. Verfahren nach einem der vorhergehenden Ansprüche, **dadurch gekennzeichnet, dass** das Protein APC11 das Humanprotein APC11 ist.

9. Verfahren nach einem der vorhergehenden Ansprüche, **dadurch gekennzeichnet, dass** die Probe, die von dem Patienten stammt, vorzugsweise vom Primärtumor stammt.

10. Verfahren nach einem der vorhergehenden Ansprüche, **dadurch gekennzeichnet, dass** der Referenzwert dem Expressionsniveau des Proteins APC11, gemessen in einer Probe, die von einer gesunden Person stammt, die nicht an Krebs leidet, entspricht.

11. Verfahren nach einem der vorhergehenden Ansprüche, **dadurch gekennzeichnet, dass** das Expressionsniveau des Proteins APC11 unter Verwendung von Antikörpern Anti-APC11 quantifiziert wird.

12. Verfahren nach Anspruch 11, **dadurch gekennzeichnet, dass** die Quantifizierung des Expressionsniveaus des Proteins APC11 durch Immunhistochemie bestimmt wird.

13. Verwendung der Messung des Expressionsniveaus von APC11 in vitro als Biomarker für die Prognose von Brust- und Dickdarmkrebs.

14. Verwendung nach Anspruch 13, **dadurch gekennzeichnet, dass** die Prognose darin besteht, die Aggressivität oder die Wahrscheinlichkeit der Entwicklung von Metastasen durch diese Krebsformen zu bestimmen.

15. Verwendung nach einem der Ansprüche 13 und 14, **dadurch gekennzeichnet, dass** die Prognose darin besteht, die Wahrscheinlichkeit der Entwicklung von Metastasen durch den Brustkrebs zu bestimmten.

16. Verwendung nach einem der Ansprüche 13 und 14, **dadurch gekennzeichnet, dass** die Prognose darin besteht, die Rezidivgefahr von Dickdarmkrebs und insbesondere die Gefahr für ein lokales Rezidiv, zu bestimmen.

**Claims**

1. A method for identifying an aggressive cancer or which may develop metastases in a patient affected with breast or colon cancer, and comprising the steps of:

   - measuring the expression level of the APC11 protein in a sample stemming from said patient, and
   - classifying the cancer as aggressive or which may develop metastases when a significant difference in the expression level of the APC11 protein is detected with respect to a reference value, said reference value may be:

   i) an expression threshold value, obtained by determining the number or the proportion of cells which express the APC11 protein or further the amount or intensity of expression of the APC11 protein within samples from patients affected with breast or colon cancer and the subsequent fate of which is known as regards aggressiveness of the cancer, and/or development of metastases,

ii) an expression level of the APC11 protein measured in a biological sample from a healthy subject or else affected with the same type of cancer but which is not aggressive or able to metastasize,

iii) an average value of the expression level of the APC11 protein which is detected on a pool of biological samples from healthy subjects or else subjects affected with the same type of cancer but which is not aggressive or able to metastasize.

2. The method according to claim 1, **characterized in that** said patient is affected with breast cancer and **in that** the cancer is classified as being capable of developing metastases when a significant reduction in the expression level of the APC11 protein is detected in the sample from said patient with respect to a reference value.

3. The method according to claim 2, **characterized in that** the reduction of the expression of the APC11 protein is at least 50%.

4. The method according to any of claims 1 to 3, **characterized in that** said patient is affected with breast cancer which had been considered with a good prognosis at the moment of the diagnosis.

5. The method according to claim 1, **characterized in that** said patient is affected with colon cancer and that the cancer is classified as aggressive when a significant increase in the expression level of APC11 is detected in the sample from said patient to be tested with respect to the reference value.

6. The method according to claim 5, **characterized in that** the cancer is classified as aggressive when the increase in the expression of APC11 is at least 50%.

7. The method according to one of claims 5 or 6, **characterized in that** said patient is affected with node-negative colon cancer.

8. The method according to any of the preceding claims, **characterized in that** said APC11 protein is human APC11 protein.

9. The method according to any of the preceding claims, **characterized in that** the sample from said patient is a tissue preferably from the primary tumor.

10. The method according to any of the preceding claims, **characterized in that** the reference value corresponds to the expression level of the APC11 protein measured in a sample from a healthy subject who is not affected with cancer.

11. The method according to any of the preceding claims, **characterized in that** the expression level of the APC11 protein is quantified by using anti-APC11 antibodies.

12. The method according to claim 11, **characterized in that** the quantification of the expression level of the APC11 protein is determined by immunochemistry.

13. The use of the measurement of the APC11 expression level in vitro as a biomarker for prognosis of breast and colon cancers.

14. The use according to claim 13, **characterized in that** the prognosis consists of determining the aggressiveness or the capability of developing metastases of said cancers.

15. The use according to one of claims 13 and 14, **characterized in that** the prognosis consists of determining the capability of developing metastases of breast cancer.

16. The use according to one of claims 13 and 14, **characterized in that** the prognosis consists of determining the risk of relapse of colon cancers and notably of local relapse.

FIG.1A

FIG.1B

A

Quantité relative d'ARN$_m$

p = 0,0181    p = 0,0226    p = 0,0435

Non métastatiques
métastatiques

Tumeur primaire
métastatiques

Epithélial
Mésenchymateux

FIG.2A

B

| M+/ E | M+/ E | M$_0$/ E-M | M$_0$/ E | M+/ M | M+/ M | M+/ M | M+/ E-M | M$_0$/ E |
|---|---|---|---|---|---|---|---|---|
| MCF-7 | T47D | BT-20 | UACC-812 | MM-157 | MM-231 | MM-549 | HBL-100 | HME-1 |

FIG.2B

APC-11

actine

FIG.2C

EP 2 764 365 B1

FIG.3

FIG.4

FIG.5

FIG.6

FIG.7

FIG.8

FIG.9

FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1158910

**Littérature non-brevet citée dans la description**

- Contribution of cell culture, RNA extraction, and reverse transcription to the measurement error in quantitative reverse transcription polymerase chain reaction-based gene expression quantification. **COMBES J. D et al.** Analytical Biochemistry. Academic Press Inc, 2009, vol. 393, 29-35
- **MICHIELS S.** *Lancet,* 2005, vol. 365 (9458), 488-492
- **CHAN, AH.** *J. Cell Biochem.,* 2001, vol. 83, 249-258
- **GAYET et al.** *Oncogene,* 2001, vol. 20 (36), 5025-5032
- **CAJOT et al.** *Cancer Res.,* 1997, vol. 57 (13), 2593-2597
- **HAMMOND ; STAMPFER.** *Proc Natl Acad Sci U S A.,* 1984, vol. 81 (17), 5435-5439
- **WANG et al.** *Oncogene,* 2003, vol. 22 (10), 1486-1490
- **CREIGHTON et al.** *Breast Cancer Res. Treat.,* 2009, vol. 114, 287-299
- **HUGH et al.** *J. Clin. Oncol.,* 2009, vol. 27, 1168-1176
- **SULLIVAN et al.** *Stat. Med.,* 1999, vol. 18, 855-888
- **GOLDSTEIN et al.** Multilevel Statistical models. Arnold Publishers, 2003
- R: A language and environment for statistical computing. *R Foundation for Statistical Computing,* 2007, ISBN 3-900051-07-0, http://www.R-project.org
- **KAPLAN ; MEIER.** *J. Am. Stat. Assoc.,* 1958, vol. 53, 457-481
- **PETO et al.** *Br. J., Cancer,* 1977, vol. 35, 1-39